# EUROPEAN PATENT APPLICATION

(11) **EP 1 127 572 A2**
(43) Date of publication of application: **29.08.2001**
(21) Application number: 01103200.0
(22) Date of filing: 12.02.2001
(51) Int. Cl.: A61K 31/352, A23L 1/30, A61P 19/02, A61P 29/00

(54) **Use of flavones for treating cycloxygenase-2 mediated diseases**

(30) Priority: 25.02.2000 US 185179 P
(71) Applicant: BASF AKTIENGESELLSCHAFT, 67056 Ludwigshafen (DE)
(72) Inventor: Wenzel, Uwe, Dr., 85354 Freising (DE); Daniel, Hannelore, Prof. Dr., 85354 Freising (DE)

(57) **Abstract**

A pharmaceutical composition for inhibiting COX-2 biosynthesis comprising a therapeutically effective amount of the compound of formula I and a pharmaceutrically acceptable carrier. 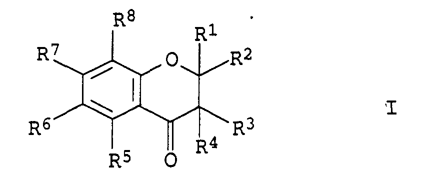 wherein
R¹ and R⁴ represent either Hydrogen or together a bond
R⁵, R⁶, R⁷, R⁸ represent independently of each other Hydrogen, Hydroxy or Methoxy; in addition R⁷ represents a sugar substituent like glucoside, rutinosid, manno gluco pyransyl, aprosylglucoside
R² and R³ represent Hydrogen, Hydroxy, Methoxy or 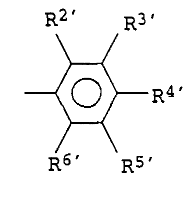 wherein R²', R³', R⁴', R⁵' and R⁶'
   are independently or each other Hydrogen, Hydroxy or Methoxy with the proviso, that R² or R³ is represented by the optionally substituted Phenylring.

## Description

The present invention encompasses flavone-type compounds useful in the treatment of cyclooxygenase-2 and NFχB mediated diseases especially arthritis and Alzheimer's disease. More particularly, this invention concerns a method of inhibiting cyclooxygenase-2 (COX-2) and NFχB.

The prostaglandins are extremely potent substances which produce a wide variety of biological effects, often in the nanomolar to picomolar concentration range. The discovery of two forms of COX, isoenzymes COX-1 and COX-2, that catalyze the oxidation of arachidonic acid leading to prostaglandin biosynthesis has resulted in renewed research to delineate the role of these two isozymes in physiology and pathophysiology. These isozymes have been shown to have different gene regulation and represent distinctly different prostaglandin biosynthesis pathways. The COX-1 pathway is expressed constitutively in most cell types. It responds to produce prostaglandins that regulate acute events in vascular homeostasis and also has a role in maintaining normal stomach and renal function. The COX-2 pathway involves an induction mechanism which has been linked to inflammation, mitogenesis and ovulation phenomena.

Prostaglandin inhibitors provide therapy for pain, fever, and inflammation, and are useful therapies, for example in the treatment or rheumatoid arthritis and osteoarthritis. The non-steroidal anti-inflammatory drugs (NSAIDs) such as ibuprofen, naproxen and fenamates inhibit both isozymes. Inhibition of the constitutive enzyme COX-1 results in gastrointestinal side effects including ulcers and bleeding and incidence of renal problems with chronic therapy. Inhibitors of the induced isozyme COX-2 may provide anti-inflammatory activity without the side effects of COX-1 inhibitors.

The Nuclear factor kappa B (NFχB) is a pivotal Transcription factor in chronic inflammatory diseases (for review see New England J. Med. 336 (1997) p. 1066). NFχB mediates inflammatory response in diseases as arthritis (osteoarthritis and rheumatoid arthritis), asthma, inflammatory bowel disease and other inflammatory diseases.

The present invention discloses flavone-compounds which are inhibitors of biosynthesis of COX-2, NFχB and both, CO2-X and NFχB.

Flavonoids are known as inhibitors of the growth of Caco 2 and HT-29 cell lines (Eur. J. Nutr. 38 (1999) p. 133; Proc. Germ. Nutr. Soc. 1 (1999) p. 28)

The compounds of the invention have the formula I 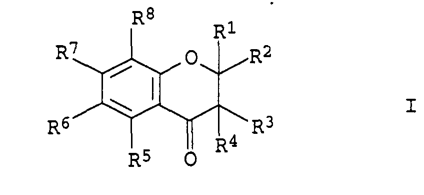 wherein
R¹ and R⁴ represent either Hydrogen or together a bond
R⁵, R⁶, R⁷, R⁸ represent independently of each other Hydrogen, Hydroxy or Methoxy; in addition R⁷ represents a sugar substituent like glucoside, rutinosid, manno gluco pyransyl, aprosylglucoside
R² and R³ represent Hydrogen, Hydroxy, Methoxy or 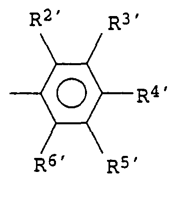 wherein R²', R³', R⁴', R⁵' and R⁶' are independently or each other Hydrogen, Hydroxy or Methoxy with the proviso, that R² or R³ is represented by the optionally substituted Phenylring and their pharmaceutical acceptable salts. Pharmaceutical acceptable salts are salts of anorganic of organic acids like HCl, H₃PO₄, H₂SO₄, H₂CO₃, Acetic acid, Citric acid and other acids well known in the state of the art of pharmaceutical substances.

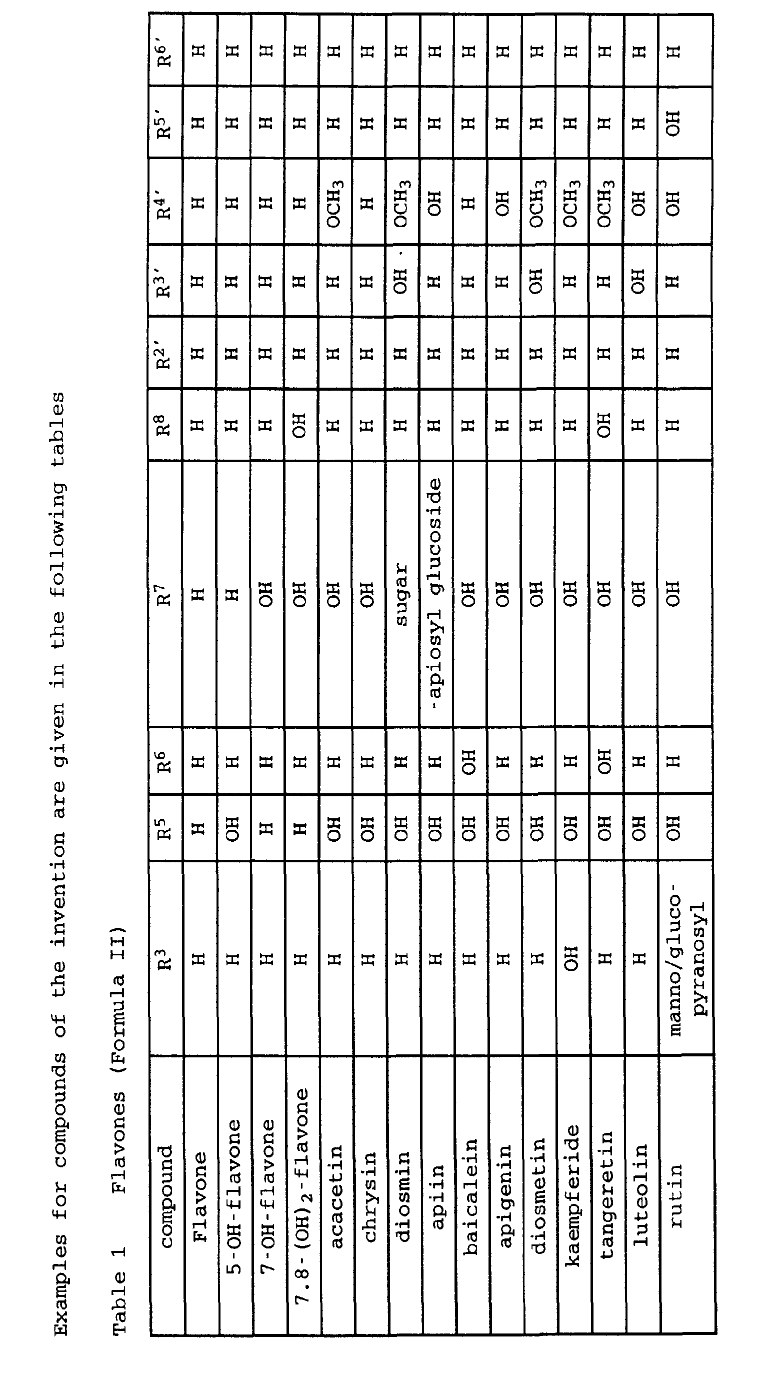

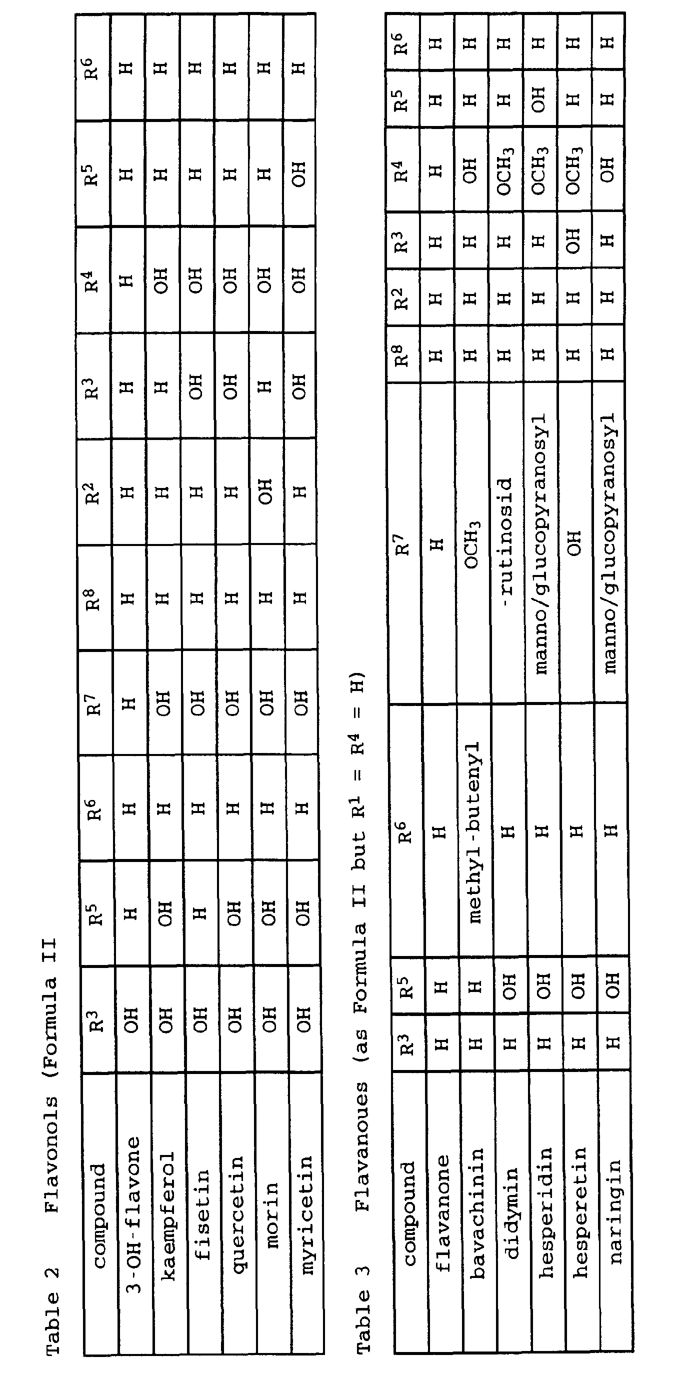

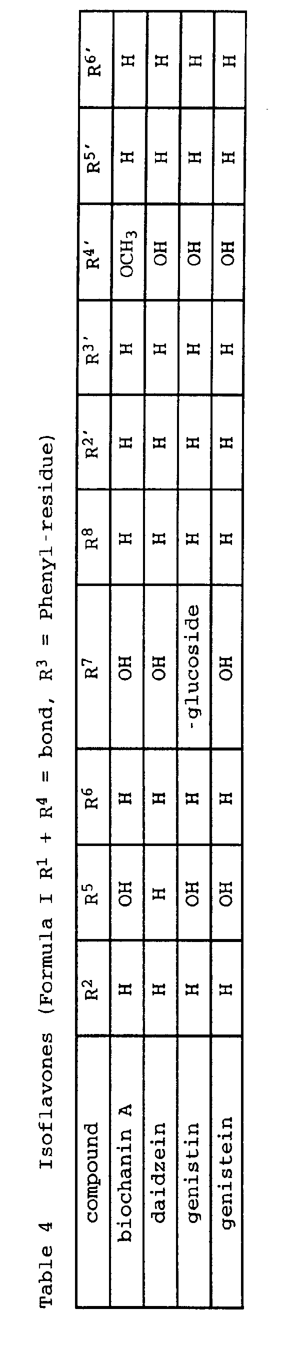

Preferred compounds of the invention have the formula II 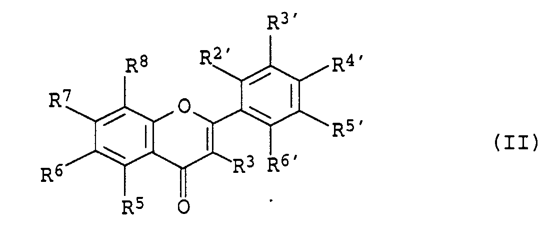 wherein R³ represents Hydrogen, Hydroxy or Methoxy and R⁵, R⁶, R⁷, R⁸, R^{2'}, R^{3'}, R^{4'}, R^{5'}, R^{6'} are as given in formula I.

Specifically preferred are compounds selected the group of:
Flavone,
Baicalein,
Diosmin,
Kaempferide
Tangeretine,
3-OH-Flavone
Fisetin,
Myricetin,
Flavanone,
Hesparidin,
Hesperetin,
Bavachinin,
Daidzein,
Genistein,
Genistin,
Luteolin,
Quercetin,
Didymin,
Biochamin.

The most preferred compound is flavone.

The compounds of the invention are inhibitors of COX-2 or NFχB or COX-2 and NFχB and can be used for the manufacture of a medicament for the treatment or prophylaxis of diseases mediated by COX-2 or COX-2 and NFχB.

The compounds of the invention inhibit the biosynthesis of COX-2 or NFχB or COX-2 and NFχB.

The following examples illustrate the invention but are not to be contrued as limitation of the invention.

### Example 1

### Cell Culture of HT-29 Cells

HT-29 cells (passage 106) were provided by ATCC and used between passage 150 and 200. Cells were cultured and passaged in RPMI-1640 supplemented with 100 % FCS and 2 mM glutamine (all from GIBCO, Eggenstein, Germany). Antibiotics added were 100 U/ml penicillin and 100 µg/ml streptomycin (GIBCO). The cultures were maintained in a humidified atmosphere of 95 % air and 5 % CO₂ at 37°C. Cells were passaged at preconfluent densities by the use of a solution containing 0.05 % trypsin and 0.5 mM EDTA (GIBCO).

### Example 2

Semi-quantitative RT-PCR

RNA from HT-29 cells was isolated at the times indicated in table 5 according to the method described by Chomczynski and Sacchi Anal. Biochem. 162 (1987) 156 with slight modifications. Reverse transcription was done with 5 µg of isolated RNA. First strand cDNA synthesis was accomplished with an oligo- (dT)₁₅ primer (MBI Fermentas). Amplification of sequence specific fragments (Taq Polymerase was from Sigma) was performed with 30 cycles (95°C denaturation for 1 min, 55°C hybridization for 2 min, 72°C extensions for 2 min; Personal Cycler, Biometra, Göttingen, Germany). RT-PCR products were separated on a 1 % agrose gel and visualized by ethidium bromide. The amount of first strand used to amplify specific sequences was derived from the linear range of amplification. The amplified GAP-DH sequence was used as a constitutively expressed control. The amplivied products were photographed and the intensity of the bands was analyzed by the SigmaGel software. No products were obtained for all genes without reverse transcription the specificity of mRNA determination. A λ-DNA/EcoRI + HindIII marker (MBI Fermentas) was used in all PCR experiments as a size control of the amplified products. Amplified cDNA sequences (primers were custom synthesized by Eurogentec, Seraing, Belgium) were GAP-DH: bp 558-1010; COX-2: bp 1366-1870, NFχB:bp 2832-3401.

To derive the EC₅₀ values for growth inhibition a non-linear approximation model by the least square methods based on a competition curve using one component was applied (GraphPadPrism, GraphPad, USA). For statistical analysis a students t-test (GraphPadPrism) was used. For each variable at least 3 independent experiments were carried out. Data are given as the mean ± SEM.

### Example 3

Flavone Effects on Gene Expression in HT-29 Cells

To assess whether the effects of flavone on cell-cycle arrest and apoptosis observed in HT-29 cells were mediated by altered gene expression, mRNA levels of genes were determinated. COX-2 was found to be diminished in its transcript levels drastically when cells were exposed to flavone for 48 h (Table 5). The mRNA of NFχB (nuclear transcription factor χB) was also reduced dramatically by flavone treatment. That this effect on gene expression occurred selectively was shown by unaltered mRNA levels of the tumor suppressor p53.

**Table 5**

| Semi-quantitative determination of COX-2 and NFχB mRNA levels in HT-29 cells by use of RT-PCR (n=4). The cells were incubated for the indicated time points with or without (control) 150 µM flavone and subsequently RNA was isolated, reversely transcribed and cDNA sequences of COX-2, NFχB and GAP-DH (as a constitutively expressed standard gene) were amplified by specific primers. | | | | |
|---|---|---|---|---|
| incubation time [h] | 3 h | 8 h | 24 h | 48 h |
| COX-2/GAPDH [AUC] control | 0.74 | 0.60 | 0.71 | 0.71 |
| 150 µM flavone | 0.69 | 0.35 | 0.24* | 0.12** |
| NFχB/GAPDH[AUC] control | 1.4 | 1.7 | 1.8 | 1.5 |
| 150 µM flavone | 1.1 | 1.7 | 0.8* | 0.4** |

| | | | | |
|---|---|---|---|---|
| * P < 0.05 | | | | |
| ** P < 0.01 | | | | |

### Pharmaceutical Compositions

The present invention also provides pharmaceutical compositions which comprise compounds of the present invention formulated together with one or more non-toxic pharmaceutically acceptable carriers. The pharmaceutical compositions of the present invention comprise a therapeutically effective amount of a compound of ' the present invention formulated together with one or more pharmaceutically acceptable carriers. As used herein, the term "pharmaceutically acceptable carrier" means a non-toxic, inert solid, semi-solid or liquid filler, diluent, encapsulating material or formulation auxiliary of any type. Some examples of materials which can serve as pharmaceutically acceptable carriers are sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower'oil; sesame oil; olive oil; Corn oil and soybean oil; glycols; such a propylene glycol; esters such as ethyl oleate and ethyl laurate; agar; buffering agents such as magnesium hydroxide and aluminium hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the procedures and judgements well known to one skilled in the art. The pharmaceutical compositions of this invention can be administered to humans and other animals orally, rectally, parenterally, intracisternally, intravaginally, intraperitoneally, topically (as by powders, ointments, or drops), bucally, or as an oral or nasal spray.

It is a further object of the present invention to provide such a therapeutic composition which is a nutraceutical - that is, a composition which includes only naturally-occurring components capable of providing beneficial therapeutic and health promoting effects.

Another object of the present invention is use of compounds of formula I or II in food for the treatment or prevention of diseases by inhibiting the prostaglandin and NFχB biosynthesis.

The compounds of the present invention may be potentially useful in the treatment of several illness or disease states such as Alzheimer's disease, cardiovascular diseases, ischemiare perfusion nijurg, inflammatory bowel diseases, immume disorders including HIV-infection, sepsis, autoimmume diseases, diabetes, inflammatory diseases, dysmennorhea, asthma, premature labor, adhesions and in particular pelvic adhesions, osteoporosis, and ankylosing spondolitis.

The compounds of the present invention may be useful by providing a pharmaceutical composition for inhibiting prostaglandin biosynthesis, COX-2 and NFχB comprising a therapeutically effective amount of a compound of formula I and a pharmaceutically acceptable carrier.

The compounds of the present invention may be useful by providing a pharmaceutical composition for inhibiting prostaglandin biosynthesis or NFχB-biosynthesis or COX-2 and NFχB-biosynthesis comprising a therapeutically effective amount of a compound of formula II and a pharmaceutrically acceptable carrier.

In addition, the compounds of the present invention may be useful by providing a method for inhibiting prostaglandin biosynthesis comprising administering to a patient in need of such treatment a therapeutically effective amount of a compound of formula I.

The compounds of the present invention may be useful by providing a method for inhibiting prostaglandin biosynthesis comprising administering to a patient in need of such treatment a therapeutical effective amount of compound of formula II.

In addition, the compounds of the present invention may be useful by providing a method for treating pain, fever, inflamation, rheumatoid arthritis, osteoarthritis and adhesions, comprising administering to a patient in need of such treatment a therapeutically effective amount of a compound of formula I.

In addition, the compounds of the present invention may be useful by providing a method for treating pain, fever, inflamation, rheumatoid arthritis, osteoarthritis and adhesions, comprising administering to a patient in need of such treatment a therapeutically effective amount of a compound of formula II.

In addition the compounds of the present invention may be useful for prevention of the above mentioned diseases. For prophylaxis of those diseases like inflammation, rheumatoid arthritis, osteoarthritis and Alzheimer's disease the compounds of the invention are used at doses lower than the therapeutical effective amount. The compounds of the invention can be used as dietary supplements, added as the active ingredients to foods or medical foods or as oral compositions.

Liquid dosage forms for oral administration include pharmaceutically acceptable emulsions, microemulsions, solutions suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (such as, for example, cottonseed, groundnut, corn, germ, olive, castor, sesame oils, and the like), glycerol, tetrahydrofurfuryl alcohol, poly-ethyl-ene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, such as, for example, sterile injectable aqueous or oleaginous suspensions may be formulated according to the known art using suitable dispersing or wetting agents and suspending agents. The sterile injectable preparation may also be a sterile injectable solution, suspension or emulsion in a non-toxic parenterally acceptable diluent or solvent, such as, for example, a solution in 1,3-butanediol. Among the acceptable vehicles and solvents that may be employed are water, Ringer's solution, isotonic sodium chloride solution, and the like. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectable preparations.

The injectable formulations can be sterilized by any method known in the art, such as, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a drug, it is often desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle. Injectable depot forms are made by forming microencapsulated matrices of the drug in biodegradable polymers such as polyactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissues.

Compositions for rectal or vaginal administration are preferably suppositories which can be prepared by mixing the compounds of this invention with suitable non-irritating excipients or carriers such as cocoa butter, polyethylene glycol or a suppository wax which are solid at ambient temperature but liquid at body temperature and thus melt in the rectum or vaginal cavity and release the active compound.

Solid dosage forms for oral administration include capsules, tablets, pills, powders, and granules. In such solid dosage forms, the active compound is usually mixed with at least one inert, pharmaceutically acceptable excipient or carrier such as, for example, sodium citrate or dicalcium phosphate and/or a) fillers or extenders such as, for example, starches, lactose, sucrose, glucose, mannitol and silicic acid, b) binders such as, for example, carboxymethylcellulose, alginates, gelatin, polyvinylpyrrolidinone, sucrose, and acacia, c) humectants such as, for example, glycerol, d) disintegrating agents such as, for example, agar-agar, calcium carbonate, potato or tapioca starch, alginic acid, certain silicates, and sodium carbonate, e) solution retarding agents such as, for example, paraffin, f) absorption accelerators such as, for example, quaternary ammonium compounds, g) wetting agents such as, for example, cetyl alcohol an glycerol monostearate, h) absorbents such as, for example, kaolin and bentonite clay, and lubricants such as, for example, talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients such as, for example, lactose or milk sugar as well as high molecular weight polyethylene glycols and the like.

Solid compositions of a similar type may also be employed as fillers in soft and hard-filled gelatin capsules using excipients such as, for example, lactose or milk sugar as well as high molecular weight polyethylene glycols and the like.

The active compounds can also be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulation art. In such solid dosage forms the active compound may be admixed with the least one inert diluent such as, for example, sucrose, lactose or starch. Such dosage forms may also comprise, as is normal practice, additional substances other than inert diluents, e.g., tableting lubricants and other tableting aids such as, for example, magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, eye ointments, powders and solutions are also contemplated as being within the scope of this invention.

The ointments, pastes, creams and gels may contain, in addition to an active compound of this invention, excipients such as, for example, animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to the compounds of this hydroxide, calcium silicates and polyamide powder, or mixtures of these substances. Sprays can additionally contain customary propellants such as chlorofluorohydrocarbons.

Transdermal patches have the added advantage of providing controlled delivery of a compound to the body. Such dosage forms can be made by dissolving or dispensing the compound in a suitable medium. Absorption enhancers can also be used to increase the flux of the compound across the skin. The rate can be controlled by either providing a rate controlling membrane or by dispersing the compound in a polymer matrix or gel.

According to the methods of treatment of the present invention, a patient, is treated by administering to the patient a therapeutically effective amount of a compound of the invention, in such amounts and for such time as is necessary to achieve the desired result. By a "therapeutically effective amount" of a compound of the invention is meant a sufficient amount of the compound to provide the relief desired, at a reasonable benefit/risk ratio applicable to any medical treatment. It will be understood, however, that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts.

The compound of the inventions can be added to nutritional substance which can be a food preparation or an essential nutrient preparation.

Essential nutrient preparations are materials which contain one or more essential nutrients. Where only one essential nutrient is present in the essential nutrient preparation, that essential nutrient can be a vitamin. As used herein, essential nutrients are those nutients which are required to sustain health but which cannot be effectively produced by humans. Examples of essential nutrients are compiled in a number of published sources, including Modern Nutrition in Health and Disease, 8th ed., Shils et al., eds., Philadelphia:Lea and Febiger (1994), which is hereby incorporated by reference. Essential nutients are meant to include essential vitamins and provitamins thereof, essential fats, essential minerals, such as those minerals for which daily values have been recommended, and essential amino acids. One example of an essential nutrient preparation is a formulation which contains a vitamin and a caloric content of less than 2.5 cal per dry gram, preferably less than 2 cal per dry gram, most preferably less than 1.8 cal per dry gram. Essential nutrient preparations also include those materials which contain at least one vitamin in an amount greater than 15%, preferably greater than 20%, more preferably greater than 40% of the U.S. adult RDA for that essential nutrient per gram of essential nutrient preparation. Still other suitable essential nutrient preparations contain at least two vitamins, each in an amount greater than 10%, preferably greater than 15%, more preferably greater than 20% of the U.S. adult RDA for that essential nutrient per gram of essential nutrient preparation. Suitable essential nutrient preparations are commonly referred to as dietary supplements, vitamin supplements, and mineral supplements, multiple vitamin supplements, and the like, and are typically available commercially in the form of pills, tablets, capsules, powders, syrups, and suspensions. Preferably, the essential nutrient composition contains at least one essential nutrient in an amount greater than 25%, more preferably greater than 50%, and most preferably greater than or equal to 100% of the daily requirement for that essential nutrient per customarily consumed quantity of the essential nutrient preparation.

As indicated above, the nutritional substance can also be a food preparation. Food preparations are materials which contain one or more amino acid, carbohydrate, or fat, which are suitable for human or animal consumption, and which are not essential nutrient preparations. It is preferred that the food preparation be a two or more component food preparation. For example, a two or more component food preparation can be a mixture of two or more one-component foods. One component foods are foods which are derived substantially from a single natural source. A small percentage of the one-component food can be derived from a second natural source, but that percentage, by weight, is preferably less than 5%, more preferably less than than 1%, more preferably less than 0.1%. One component foods include, for example, sugar, unsweetened juice, nectar, or puree from a single species of plant, such as unsweetened apple juice (including a blend of different varieties of apple juice), grapefruit juice, orange juice, apple sauce, apricot nectar, tomato juice, tomato sauce, tomato puree, and the like. Grain plants of a single species and materials produced from grain plants of a single species, such as corn syrup, rye flour, wheat flour, oat bran, and the like are also considered to be one component foods. Alternatively, the two or more component food preparation can be a mixture of one or more one component foods and one or more essential nutrients. Preferably, the amount of at least one of the one or more essential nutrients present in the two component food is greater than the amount of the at least one essential nutrient that is naturally present collectively in the one or more one component foods. For example, where the essential nutrients are vitamin X and vitamin Y and where the one component food is orange juice and where the orange juice naturally contains vitamin X and vitamin Y in amounts "Nx" and "Ny", respectively, it is preferred that the composition contain vitamin X and vitamin Y in amounts "Tx" and "Ty", respectively, so that Tx is greater than Nx, Ty is greater than Ny, or both.

Food preparations particularly well suited to the practice of the present invention include breakfast foods, such as prepared cereals, toaster pastries, and breakfast drink mixes; infant formulas; dietary supplements; complete diet formulas; and weight-loss preparations, such as weight-loss drinks and weight-loss bars.

The food preparation can be one which naturally contains no compounds of formula I or II. Alternatively, it can contain a natural molar amount of a natural isomer of. For purposes of this application, the molar amount of natural compounds of formula I or II contained per gram of food is designated "N". For some foods, the molar amount of compounds of formula I or II naturally present is known. For others, the molar amount of compounds of formula I or II can be determined by a number of sensitive and specific methods, such as high-performance liquid chromatography ("HPLC") and gas chromatography ("GC").

The molar amount of natural compounds of formula I or II present in the composition of the present invention is greater that the molar amount of present in the food preparation. For purposes of this application, the molar amount of natural isomer of compounds of formula I or II contained per gram of composition is designated "T". Thus, T necessarily must be greater than N. Preferably, T/N is greater than 105%; more preferably, it is greater that 110%; and, most preferably, it is greater than 120%.

As indicated above, the composition can, optionally, include one or more unnatural isomers of compounds of formula I or II. When present in the composition, the one or more unnatural isomers of is preferably present in a molar amount which is less than T minus N.

The present invention also relates to a method for increasing the content of compounds of formula I or II of a nutritional substance. The nutritional substance can be a food preparation, an essential nutrient preparation, or a combination of a food preparation and an essential nutrient preparation. The content compounds of formula I or II of the nutritional substance is increased by incorporating one or more natural isomers of compounds of formula I or II into or with the nutritional substance. This can be achieved by methods well known in the art of food and essential nutrient preparation, such as by homegenizing, coating, spraying, coarsely mixing, tossing, kneading, pilling, and extruding one or more, singly or in combination, onto or with the nutritional substance.

One or more of the one or more compounds of formula I or II that are added to the nutritional substance in accordance with the present invention can be substantially chirally pure or each of the one or more compounds of formula I or II can be chirally pure. Alternatively, one or more of the one or more natural compounds of formula I or II can be present in a mixture with one or more unnatural compounds of formula I or II. The molar amount of the one or more natural compounds of formula I or II and the one or more unnatural compounds of formula I or II present in the mixture added to the nutritional substance can be the same, as in the case where a racemic mixture is added, or they can be different. Preferably the molar amount of the natural isomer exceeds the molar amount of the unnatural isomer. Additionally or alternatively, unnatural isomer can be incorporated in a separate step subsequent to or prior to incorporating the one or more natural isomers into the nutritional substance. It is preferred that the collective molar amount of unnatural isomer added before, during, and/or after the addition of natural isomer be less than the collective molar amount of natural isomer added.

Natural isomers of reduced folates that are substantially chirally pure can be prepared by any suitable method, including, for example, by classical chemical synthesis or increasing by biotechnology means of the amount of compounds of formula I or II in plants used for preparation of nutritional substance.

The compositions of the present invention can be used to increase a subject's dietary intake of compounds of formula I or II by administering the composition to the subject. Certain classes of individuals are viewed to be especially benefitted by increasing dietary intake of compounds of formula I or II. These include pregnant females; females who have had a miscarriage; females who have carried a fetus having a neural tube defect, a cleft lip defect, or a cleft palate defect; and humans who suffer vascular disease.

EXAMPLES for
1) A typical ready to eat breakfast cereal: corn (and/or other grains), sugar, salt, malt flavoring, such that a 30 g serving provides about 2 g of protein, 26 g total carbohydrate, and 330 mg of sodium, also containing per serving size vitamin A palmitate (15% of RDI), ascorbic acid (25% of RDI), reduced iron (45% of RDI), vitamin D (10% of RDI), thiamin hydrochloride (25% of RDI), riboflavin (25% of RDI), niacinamide (25% of RDI), pyridoxine hydrochloride (25% of RDI), and 100 mg of flavone.
2) A typical daily multivitamin tablet: calcium carbonate, ascorbic acid (60 mg, 100% RDI), gelatin, vitamin E acetate (30 I.U., 100% RDI), starch, niacinamide (20 mg, 100% RDI), hydroxypropyl-methylcellulose, calcium pantothenate (10 mg, 100% RDI), calcium silicate, hydroxypropylcellulose, pyridoxine hydrochloride (2 mg, 100% RDI), riboflavin (1.7 mg, 100% RDI), thiamin mononitrate (1.5 mg, 100% RDI), beta carotene & vitamin A acetate (5000 I.U., 100% RDI), sodium hexametaphosphate, magnesium stearate, vitamin D (400 I.U., 100% RDI), vitamin B,.sub.2 (6 .mu.g, 100% RDI), lecithin, and 700 mg of flavone.
3) A typical daily multivitamin and minerals tablet: calcium phosphate (130 mg of elemental calcium), magnesium hydroxide & stearate (100 mg, 25% RDI), cellulose, potassium chloride, ascorbic acid (60 mg, 100% RDI), gelatin,ferrous fumarate (18 mg elemental iron, 100% RDI), zinc sulfate (15 mg, 100% RDI), modified cellulose gum, vitamin E acetate (30 I.U., 100% RDI), citric acid, niacinamide (20 mg, 100% RDI), magnesium stearate, hydroxypropyl-methylcellulose, calcium pantothenate (10 mg, 100% RDI), selenium yeast, polyvinylpyrrolidone, hydroxypropylcellulose, manganese sulfate, silica, copper oxide (2 mg, 100% RDI), chromium yeast, molybdenum yeast, pyridoxine hydrochloride (2 mg, 100% RDI), riboflavin (1.7 mg, 100% RDI), thiamin mononitrate (1.5 mg, 100% RDI), beta carotene & vitamin A acetate (5000 I.U., 100% RDI), potassium iodide (150 .mu.g, 100% RDI), sodium hexametaphosphate, biotin (30 .mu.g, 10% RDI), vitamin D (400 I.U., 100% RDI), vitamin B.sub.12 (6 .mu.g, 100% RDI), lecithin, and 700 mg of flavone.
4) A typical daily multivitamin and minerals tablet for older adults: calcium carbonate, calcium phosphate (200 mg Ca, 20% RDI; 48 mg phosphorous, 5% RDI), magnesium oxide, magnesium stearate (100 mg, 25% RDI), potassium chloride (80 mg, 2% RDI), microrystalline cellulose, ascorbic acid (60 mg, 100% RDI), gelatin, d 1-alfa-tocopheryl acetate (45 I.U., 150% RDI), modified food starch, maltodextrin, crospovidone, reduced iron (4 mg, 22 RDI), hydroxypropyl methylcellulose, niacinamide (20 mg, 100% RDI), zinc oxide (15 mg, 100% RDI), calcium pantothenate, manganese sulfate (3.5 mg), vitamin D (400 I.U., 100% RDI), titanium dioxide, vitamin A and beta.-carotene (5000 I.U., 100% RDI), stearic acid, pyridoxine hyrochloride (3 mg, 150% RDI), riboflavin (1.7 mg, 100% RDI), silicon dioxide, copper oxide (2 mg, 100% RDI), dextrose, thiamin mononitrate (1.5 mg, 100% RDI), triethyl citrate, polysorbate 80, chhromium chloride (130 .mu.g), artificial colors, potassium iodide ((150 .mu.g, 100% RDI), sodium metasilicate (2 mg), sodium molybdate (160 .mu.g), borates, sodium selenate (20 .mu.g), biotin (30 .mu.g, 10% RDI), sodium metavanadate (10 .mu.g), cyanocobalamin (25 .mu.g, 417% RDI), nickelous sulfate (5 .mu.g), and phytonadione, and 700 mg of flavone.
5) A typical complete diet drink: water, sugar, calcium and sodium caseinates, maltodextrin, high-oleic safflower oil, soy protein, soy oil, canola oil, cocoa, sodium and potassium citrates, calcium carbonate and phosphate (250 mg Ca, 25% RDI), magnesium chloride and phosphate (100 mg Mg, 25% RDI), sodium chloride, soy lecithin, choline chloride, flavor, ascorbic acid (30 mg, 50% RDI), carrageenan, zinc sulfate (5.6 mg, 37% RDI), ferrous sulfate (4.5 mg Fe, 25% RDI), alfa-tocopheryl acetate (11.3 I.U., 37.7% RDI), niacinamide (5 mg, 25% RDI), calcium pantothenate (2.5 mg, 25% RDI), manganese sulfate (1.3 mg), copper salt (25% RDI), vitamin A palmitate (1250 I.U., 25% RDI), thiamin hydrochloride (0.375 mg, 25% RDI), pyridoxine hydrochloride (0.5 mg, 25% RDI), riboflavin (0.425 mg, 25% RDI), biotin (75 .mu.g, 25% RDI), sodium molybdate (38 .mu.g), chromium chloride (25 .mu.g), potassium iodide (37.5 .mu.g, 25% RDI), sodium selenate (18 .mu.g), phylloquinone (vitamin K.sub.1), cyanocobalamin (1.5 .mu.g, 25% RDI), vitamin D.sub.3 (100 I.U., 25% RDI), and 700 mg of flavone.
6) A typical soy based infant formula: 75.5% water; 13% sucrose; 6.6% oleo oil: coconut, high oleic (safflower or sunflower), and soybean oils; 3.8% soy protein isolate; (protein 2.7 g, fat 5.3 g, carbohydrate 10.2, linoleic acid 500 mg); potassium citrate and bicarbonate (potassium 105 mg); monobasic potassium and dibasic calcium phosphates (phosphorous 63 mg); soy lecithin; taurine; calcium carrageenan; calcium hydroxide, chloride and citrate (calcium 90 mg); sodium chloride (sodium 30 mg); L-methionine; zinc (Zn 0.8 mg), ferrous (Fe 1.8 mg), and manganese (Mn 30 .mu.g) sulfates; copper salt (Cu 70 .mu.g); taurine; L-carmiting; potassium iodide (I 9 .mu.g); ascorbic acid (8.3 mg); choline chloride; alpha-tocopheryl acetate (1.4 I.U.); niacinamide (750 .mu.g); vitamin A palmitate and beta-carotene (300 I.U.); calcium pantothenate (450 .mu.g); thiamin hydrochloride (100 .mu.g); riboflavin (150 .mu.g); pyridoxine hydrochloride (62.5 .mu.g); vitamin K.sub.1 (15 .mu.g); biotin (5.5 .mu.g); vitamin D.sub.3 (60 I.U.); cyanocobalamin (0.3 .mu.g); 10 mg of flavone.

The total daily dose of the compounds of this invention administered to a human in single or in divided doses can be in amounts, for example, from 0.05 to about 500 mg/kg body weight daily or more preferably from about 1 to about 150 mg/kg body weight for oral administration or 0.01 to about 10 mg/kg for parenteral administration daily. Single dose compositions may contain such amounts or submultiples thereof to make up the daily dose.

The amount of active ingredient that may be combined with the carrier materials to produce a single dosage form will vary depending upon the host treated and the particular mode of administration.

One skilled in the art will readily appreciate that the present invention is well adapted to carry out the objects and obtain the ends and advantages mentioned. While the above description contains many specificities, these should not be construed as limitations on the scope of the invention, but rather as an exemplification of preferred embodiments thereof. Changes therein and other uses will occur to those skilled in the art which are encompassed within the spirit of the invention as defined by the scope of the claims and their legal equivalents.

## Claims

1. Use of a compound of formula I 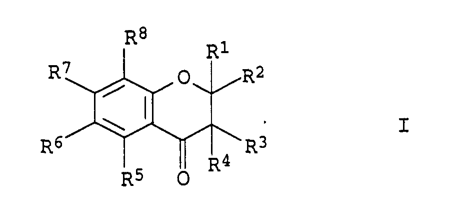 wherein
R¹ and R⁴ represent either Hydrogen or together a bond
R⁵, R⁶, R⁷, R⁸ represent independently of each other Hydrogen, Hydroxy or Methoxy; in addition R⁷ represents a sugar substituent like glucoside, rutinosid, manno gluco pyransyl, aprosylglucoside
R² and R³ represent Hydrogen, Hydroxy, Methoxy or 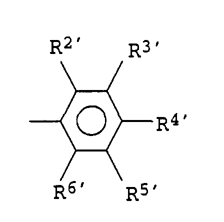 wherein R²', R³', R⁴', R⁵' and R⁶' are independently or each other Hydrogen, Hydroxy or Methoxy with the proviso, that R² or R³ is represented by the optionally substituted Phenylring and their pharmaceutrically acceptable salt for the manufacture of a medicament for the treatment or prophylaxis of diseases mediated by COX-2 or COX-2 and NFχB.

2. Use of claim 1 wherein the compound is a compound of formula II 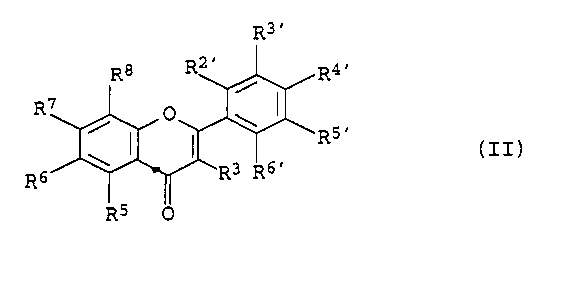 wherein R³ represents Hydrogen, Hydroxy or Methoxy and R⁵, R⁶, R⁷, R⁸, R^{2'}, R^{3'}, R^{4'}, R^{5'}, R^{6'} are as given in claim 1 and a pharmaceutical acceptable carrier.

3. Use of one of the claims 1 or 2 wherein the biosynthesis of COX-2 or COX-2 and NFχB is inhibited.

4. Use of claim 1 wherein the disease is inflammation, rheumatoid arthritis, osteoarthritis.

5. Use of one of the claims 1 to 3 wherein the disease is pain, fever, inflammation, rheumatoid arthritis, osteoarthritis, adhesions, cardiovascular diseases, Alzheimer's disease, sepsis, diabetes.

6. Use of one of the claims 1 to 5 wherein the medicament is provided as an oral composition or as nutrient preparation.

7. Food enriched with a compound of formula I of claim 1.

8. Food enriched with a compound of formula II of claim 2.

9. Food enriched with flavone.

10. Nutritional substance comprising a compound of formula II of claim 2.

11. Nutritional substance comprising flavone.
